# EUROPÄISCHE PATENTANMELDUNG

(11) **EP 1 616 870 A1**
(43) Veröffentlichungstag der Anmeldung: **18.01.2006**
(21) Anmeldenummer: 05018654.3
(22) Anmeldetag: 03.07.2002
(51) Int. Cl.: C07D 487/04, A01N 43/653

(54) **Fungizide Triazolopyrimidine, Verfahren zu ihrer Herstellung und ihre Verwendung zur Bekämpfung von Schadpilzen sowie sie enthaltende Mittel**

(30) Priorität: 05.07.2001 DE 10132059
(62) Teilanmeldung aus: 02758297.2
(71) Anmelder: BASF Aktiengesellschaft, 67056 Ludwigshafen (DE)
(72) Erfinder: Müller, Bernd, Dr., 67227 Frankenthal (DE); Sauter, Hubert, Dr., 68167 Mannheim (DE); Gewehr, Markus, Dr., 56288 Kastellaun (DE); Grammenos, Wassilios, Dr., 67071 Ludwigshafen (DE); Tormo i Blasco, Jordi, Dr., 69514 Laudenbach (DE); Grote, Thomas, Dr., 67157 Wachenheim (DE); Gypser, Andreas, Dr., 68159 Mannheim (DE); Rheinheimer, Joachim, Dr., 67063 Ludwigshafen (DE); Rose, Ingo, Dr., 68159 Mannheim (DE); Schäfer, Peter, Dr., 67308 Ottersheim (DE); Schieweck, Frank, Dr., 67258 Hessheim (DE); Rack, Michael, Dr., 69123 Heidelberg (DE); Lorenz, Gisela, Dr., 67434 Neustadt (DE); Strathmann, Siegfried, Dr., 67117 Limburgerhof (DE); Ammermann, Eberhard, Dr., 64646 Heppenheim (DE); Stierl, Reinhard, Dr., 67251 Freinsheim (DE)

(57) **Zusammenfassung**

Triazolopyrimidine der Formel I, 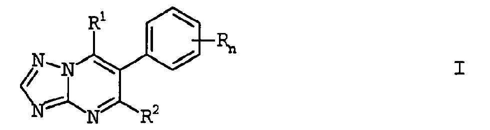 in der der Index und die Substituenten folgende Bedeutung haben:
- n: 0 oder eine ganze Zahl von 1 bis 5;
- R: Halogen, Cyano, Hydroxy, Cyanat, Alkyl, Alkenyl, Alkinyl, Halogenalkyl, Halogenalkenyl, Alkoxy, Alkenyloxy, Alkinyloxy, Halogenalkoxy, Cycloalkyl, Cycloalkenyl, Cycloalkoxy, Alkoxycarbonyl, Alkenyloxycarbonyl, Alkinyloxycarbonyl, Aminocarbonyl, Alkylaminocarbonyl, Dialkylaminocarbonyl, Alkoximinoalkyl, Alkenyloximinocarbonyl, Alkinyloximinoalkyl, Alkylcarbonyl, Alkenylcarbonyl, Alkinylcarbonyl, Cycloalkylcarbonyl, oder ein fünf- bis zehngliedriger gesättigter, partiell ungesättigter oder aromatischer Heterocyclus, enthaltend ein bis vier Heteroatome aus der Gruppe O, N oder S;
- R¹: ein fünf- bis zehngliedriger gesättigter, partiell ungesättigter oder aromatischer Heterocyclus, enthaltend ein bis vier Heteroatome aus der Gruppe O, N oder S,
wobei R und/oder R¹ gemäß der Beschreibung substituiert sein können;
- R²: Alkyl, Alkenyl oder Alkinyl, die durch Halogen, Cyano, Nitro, Alkoxy oder Alkoxycarbonyl substituiert sein können;

Verfahren zur Herstellung dieser Verbindungen, sie enthaltende Mittel sowie ihre Verwendung zur Bekämpfung von Schadpilzen.

## Beschreibung

Die vorliegende Erfindung betrifft Triazolopyrimidine der Formel I, 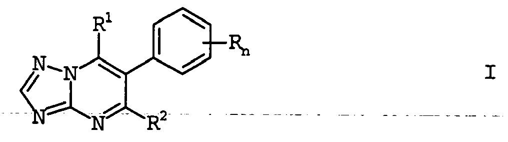
in der der Index und die Substituenten folgende Bedeutung haben:
- n: 0 oder eine ganze Zahl von 1 bis 5;
- R: Halogen, Cyano, Hydroxy, Cyanato (OCN), C₁-C₈-Alkyl, C₂-C₁₀-Alkenyl, C₂-C₁₀-Alkinyl, C₁-C₆-Halogenalkyl, C₂-C₁₀-Halogenalkenyl, C₁-C₆-Alkoxy, C₂-C₁₀-Alkenyloxy, C₂-C₁₀-Alkinyloxy, C₁-C₆-Halogenalkoxy, C₃-C₆-Cycloalkyl, C₃-C₆-Cycloalkenyl, C₃-C₆-Cycloalkoxy, C₁-C₈-Alkoxycarbonyl, C₂-C₁₀-Alkenyloxycarbonyl, C₂-C₁₀-Alkinyloxycarbonyl, Aminocarbonyl, C₁-C₈-Alkylaminocarbonyl, Di-(C₁-C₈-)alkylaminocarbonyl, C₁-C₈-Alkoximinoalkyl, C₂-C₁₀-Alkenyloximinocarbonyl, C₂-C₁₀-Alkinyloximinoalkyl, C₁-C₈-Alkylcarbonyl, C₂-C₁₀-Alkenylcarbonyl, C₂-C₁₀-Alkinylcarbonyl, C₃-C₆-Cycloalkylcarbonyl, oder ein fünf- bis zehngliedriger gesättigter, partiell ungesättigter oder aromatischer Heterocyclus, enthaltend ein bis vier Heteroatome aus der Gruppe O, N oder S;
- R¹: ein fünf- bis zehngliedriger gesättigter, partiell ungesättigter oder aromatischer Heterocyclus, ausgewählt aus 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-3-yl, 3,4-Dihydropyrazol-4-yl, 3,4-Dihydropyrazol-5-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl; 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;
wobei in den 5-Ring Heteroarylgruppen zwei benachbarte Kohlenstoffringglieder oder ein Stickstoff- und ein benachbartes Kohlenstoffringglied durch eine Buta-1,3-dien-1,4-diylgruppe verbrückt sein können;
wobei R und/oder R¹ partiell oder vollständig halogeniert oder durch eine bis vier gleiche oder verschiedene Gruppen R^{a} substituiert sein können:
- R^{a}: Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy, C₃-C₆-Alkinyloxy, C₃-C₆-Cycloalkyl, C₁-C₈-Alkoximino, C₂-C₁₀-Alkenyloximino, C₂-C₁₀-Alkinyloximino, Aryl-C₁-C₈-alkyloximino, C₂-C₁₀-Alkinyl, C₂-C₁₀-Alkenyloxycarbonyl, C₂-C₁₀-Alkinyloxycarbonyl, Phenyl, Naphthyl, fünf- bis zehngliedriger gesättigter, partiell ungesättigter oder aromatischer Heterocyclus, enthaltend ein bis vier Heteroatome aus der Gruppe O, N oder S,
wobei diese aliphatischen, alicyclischen oder aromatischen Gruppen ihrerseits partiell oder vollständig halogeniert sein oder eine bis drei Gruppen R^{b} tragen können:
- R^{b}: Halogen, Cyano, Nitro, Hydroxy, Mercapto, Amino, Carboxyl, Aminocarbonyl, Aminothiocarbonyl, Alkyl, Haloalkyl, Alkenyl, Alkenyloxy, Alkinyloxy, Alkoxy, Halogenalkoxy, Alkylthio, Alkylamino, Dialkylamino, Formyl, Alkylcarbonyl, Alkylsulfonyl, Alkylsulfoxyl, Alkoxycarbonyl, Alkylcarbonyloxy, Alkylaminocarbonyl, Dialkylaminocarbonyl, Alkylaminothiocarbonyl, Dialkylaminothiocarbonyl, wobei die Alkylgruppen in diesen Resten 1 bis 6 Kohlenstoffatome enthalten und die genannten Alkenyl- oder Alkinylgruppen in diesen Resten 2 bis 8 Kohlenstoffatome enthalten;
und/oder einen bis drei der folgenden Reste:
Cycloalkyl, Cycloalkoxy, Heterocyclyl, Heterocyclyloxy, wobei die cyclischen Systeme 3 bis 10 Ringglieder enthalten; Aryl, Aryloxy, Arylthio, Aryl-C₁-C₆-alkoxy, Aryl-C₁-C₆-alkyl, Hetaryl, Hetaryloxy, Hetarylthio, wobei die Arylreste vorzugsweise 6 bis 10 Ringglieder, die Hetarylreste 5 oder 6 Ringglieder enthalten, wobei die cyclischen Systeme partiell oder vollständig halogeniert oder durch Alkyl- oder Haloalkylgruppen substituiert sein können; und
- R²: C₁-C₄-Alkyl, C₂-C₄-Alkenyl oder C₂-C₄-Alkinyl, die durch Halogen, Cyano, Nitro, C₁-C₂-Alkoxy oder C₁-C₄-Alkoxycarbonyl substituiert sein können.

Außerdem betrifft die Erfindung Verfahren zur Herstellung dieser Verbindungen, sie enthaltende Mittel sowie ihre Verwendung zur Bekämpfung von Schadpilzen.

Aus EP-A 71 792, EP-A 550 113, WO-A 94/20501, EP-A 834 513, WO-A 98/46608 und WO-A 99/41255 sind 5-Chlortriazolopyrimidine zur Bekämpfung von Schadpilzen bekannt.

Ihre Wirkung ist jedoch in vielen Fällen nicht zufriedenstellend. Daher lag als Aufgabe zugrunde, Verbindungen mit verbesserter Wirksamkeit zu finden.

Demgemäß wurden die eingangs definierten Verbindungen gefunden. Desweiteren wurden Verfahren zu ihrer Herstellung, sie enthaltende Mittel sowie Verfahren zur Bekämpfung von Schadpilzen unter Verwendung der Verbindungen I gefunden.

Die Verbindungen der Formel I unterscheiden sich von den aus den oben genannten Schriften in der Kombination des 5-Alkylrestes mit über Kohlenstoff gebundenen Gruppen in der 7-Position.

Die Verbindungen der Formel I weisen eine gegenüber den bekannten Verbindungen erhöhte Wirksamkeit gegen Schadpilze auf.

Die Verbindungen I können auf verschiedenen Wegen erhalten werden; vorteilhaft geht man von 5-Aminotriazol der Formel II aus, das mit Dicarbonylverbindungen der Formel III kondensiert wird.

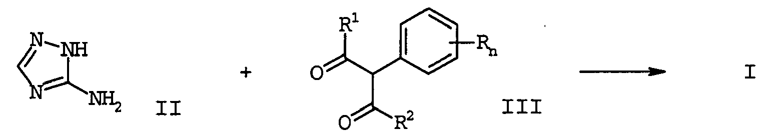

Diese Umsetzung erfolgt üblicherweise bei Temperaturen von 80°C bis 250°C, vorzugsweise 120°C bis 180°C, ohne Solvens oder in einem inerten organischen Lösungsmittel in Gegenwart einer Base [vgl. EP-A 770 615] oder in Gegenwart von Essigsäure unter den aus Adv. Het. Chem. Bd. 57, S. 81ff. (1993) bekannten Bedingungen.

Geeignete Lösungsmittel sind aliphatische Kohlenwasserstoffe, aromatische Kohlenwasserstoffe wie Toluol, o-, m- und p-Xylol, halogenierte Kohlenwasserstoffe, Ether, Nitrile, Ketone, Alkohole, sowie N-Methylpyrrolidon, Dimethylsulfoxid, Dimethylformamid und Dimethylacetamid. Besonders bevorzugt wird die Umsetzung ohne Lösungsmittel oder in Chlorbenzol, Xylol, Dimethylsulfoxid, N-Methylpyrrolidon durchgeführt. Es können auch Gemische der genannten Lösungsmittel verwendet werden.

Als Basen kommen allgemein anorganische Verbindungen wie Alkalimetall- und Erdalkalimetallhydroxide, Alkalimetall- und Erdalkalimetalloxide, Alkalimetall- und Erdalkalimetallhydride, Alkalimetallamide, Alkalimetall- und Erdalkalimetallcarbonate sowie Alkalimetallhydrogencarbonate, metallorganische Verbindungen, insbesondere Alkalimetallalkyle, Alkylmagnesiumhalogenide sowie Alkalimetall- und Erdalkalimetallalkoholate und Dimethoxymagnesium, außerdem organische Basen, z.B. tertiäre Amine wie Trimethylamin, Triethylamin, Tri-isopropylethylamin, Tributylamin und N-Methylpiperidin, N-Methylmorpholin, Pyridin, substituierte Pyridine wie Collidin, Lutidin und 4-Dimethylaminopyridin sowie bicyclische Amine in Betracht. Besonders bevorzugt werden tertiäre amine wie Tri-isopropylethylamin, Tributylamin, N-Methylmorpholin oder N-Methylpiperidin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, die Base und das Diketon III in einem Überschuß bezogen auf II einzusetzen.

Die erfindungsgemäßen Verbindungen der Formel I' sind auch zugänglich durch Umsetzung von 5-Halogentriazolopyrimidinen der Formel IV mit substituierten Malonsäureestern der Formel V, in der R^{X} für C₁-C₄-Alkyl, Allyl, Phenyl oder Benzyl steht, anschließender Verseifung des entstandenen Esters VI und Decarboxylierung der Carbonsäure VIa.

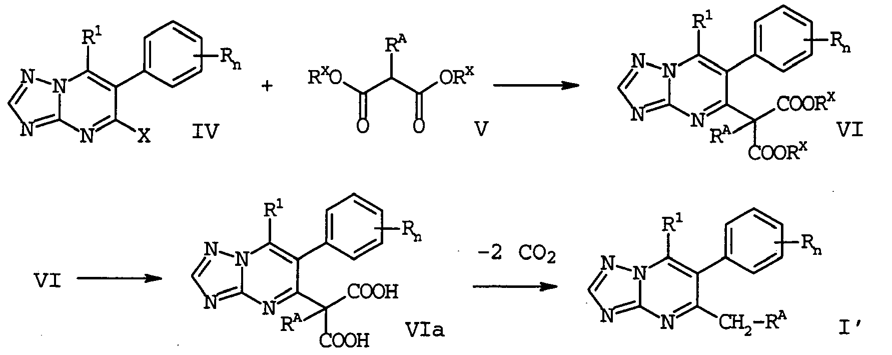

In Formel IV steht X für Halogen, insbesondere für Chlor oder Brom. Die Verbindungen IV sind aus den eingangs zitierten Schriften bekannt. In Formel I' haben n, R und R¹ die für Formel I definierte Bedeutung und R^{A} steht für Wasserstoff oder C₁-C₃-Alkyl, das durch Halogen, Cyano, Nitro oder C₁-C₂-Alkoxy substituiert sein kann.

In einer bevorzugten Ausführungsform des erfindungsgemäßen Verfahrens bedeutet R^{A} Wasserstoff oder Methyl, insbesondere Wasserstoff.

Die Ausgangsstoffe V sind in der Literatur bekannt [J. Am. Chem. Soc., Bd. 64, 2714 (1942); J. Org. Chem., Bd. 39, 2172 (1974); Helv. Chim. Acta, Bd. 61, 1565 (1978)] oder können gemäß der zitierten Literatur hergestellt werden.

Die anschließende Spaltung des Esters erfolgt unter den allgemein üblichen Bedingungen [vgl.: Greene & Wuts, Protective Groups in Organic Synthesis, Wiley (1991), S. 224 ff: Spaltung von Alkylestern unter Pd-Katalyse (S. 248); hydrierende Spaltung von Benzylestern (S. 251); Spaltung von Methyl- bzw. Ethylestern in Gegenwart von Lithiumsalzen, wie LiI (S.232), LiBr oder LiCl; oder unter sauren oder alkalischen Bedingungen]. In Abhängigkeit der Strukturelemente R^{A}, Rₙ und R¹ kann die alkalische oder die saure Verseifung der Verbindungen VI vorteilhaft sein. Unter den Bedingungen der Esterverseifung kann die Decarboxylierung zu I' bereits ganz oder teilweise erfolgen.

Die Decarboxylierung erfolgt üblicherweise bei Temperaturen von 20°C bis 180°C, vorzugsweise 50°C bis 120°C, in einem inerten Lösungsmittel, gegebenenfalls in Gegenwart einer Säure.

Geeignete Säuren sind Salzsäure, Schwefelsäure, Phosphorsäure, Ameisensäure, Essigsäure, p-Toluolsulfonsäure. Geeignete Lösungsmittel sind Wasser, aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt wird die Reaktion in Salzsäure oder Essigsäure durchgeführt. Es können auch Gemische der genannten Lösungsmittel verwendet werden.

Verbindungen der Formel I können auch durch Kupplung von 5-Halogentriazolopyrimidinen der Formel IV mit metallorganischen Reagenzien der Formel VII erhalten werden. In einer Ausführungsform dieses Verfahrens erfolgt die Umsetzung unter Übergangsmetallkatalyse, wie Ni- oder Pd-Katalyse.

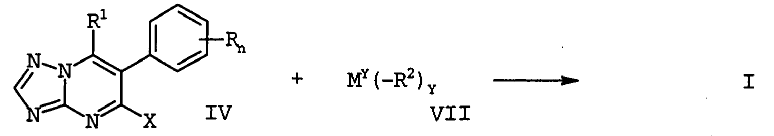

In Formel VII steht M für ein Metallion der Wertigkeit Y, wie beispielsweise B, Zn oder Sn. Diese Reaktion kann beispielsweise analog folgender Methoden durchgeführt werden: J. Chem. Soc. Perkin Trans. 1, 1187 (1994), ebenda 1, 2345 (1996); WO-A 99/41255; Aust. J. Chem., Bd. 43, 733 (1990); J. Org. Chem., Bd. 43, 358 (1978); J. Chem. Soc. Chem. Commun. 866 (1979); Tetrahedron Lett., Bd. 34, 8267 (1993); ebenda, Bd. 33, 413 (1992).

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Sofern einzelne Verbindungen I nicht auf den voranstehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl**: gesättigte, geradkettige oder verzweigte Kohlenwasserstoffreste mit 1 bis 4, 6, 8 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl:** geradkettige oder verzweigte Alkylgruppen mit 1 bis 10 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkenyl:** ungesättigte, geradkettige oder verzweigte Kohlenwasserstoffreste mit 2 bis 4, 6, 8 oder 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1propenyl und 1-Ethyl-2-methyl-2-propenyl;
**Halogenalkenyl:** ungesättigte, geradkettige oder verzweigte Kohlenwasserstoffreste mit 2 bis 10 Kohlenstoffatomen und einer Doppelbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkinyl**: geradkettige oder verzweigte Kohlenwasserstoffgruppen mit 2 bis 4, 6, 8 oder 10 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Cycloalkyl:** mono- oder bicyclische, gesättigte Kohlenwasserstoffgruppen mit 3 bis 6, 8, 10 oder 12 Kohlenstoffringgliedern, z.B. C₃-C₈-Cycloalkyl wie Cyclopropyl, Cyclobutyl, Cyclopentyl, Cyclohexyl, Cycloheptyl und Cyclooctyl, oder C₇-C₁₂-Bicycloalkyl;
**Aryl:** ein ein- bis dreikerniges aromatisches Ringsystem enthaltend 6 bis 14 Kohlenstoffringglieder, z.B. Phenyl, Naphthyl und Anthracenyl;
Außerdem werden Verbindungen I besonders bevorzugt, in denen R¹ für C₃-C₆-Cycloalkyl steht, welches durch C₁-C₄-Alkyl substituiert sein kann.

Verbindungen I werden besonders bevorzugt, in denen R^{a} für Halogen, Cyano, C₁-C₆-Alkyl, C₂-C₆-Alkenyl, C₂-C₆-Alkinyl, C₁-C₆-Alkoxy, C₁-C₆-Alkoxycarbonyl, C₁-C₆-Alkoximino, C₂-C₆-Alkenyloximino oder C₂-C₆-Alkinyloximno steht.

Insbesondere werden Verbindungen I bevorzugt, in denen R^{b} für Halogen, Cyano, C₁-C₆-Alkyl, C₂-C₆-Alkenyl, C₂-C₆-Alkinyl oder C₁-C₆₋Alkoxy steht.

Besonders bevorzugt werden auch Verbindungen I, in denen R² C₁-C₄₋Alkyl bedeutet, das durch Halogen substituiert sein kann.

Gleichermaßen besonders bevorzugt sind Verbindungen I, in denen R² für Methyl steht.

Daneben werden Verbindungen I besonders bevorzugt, in denen R² für Halogenmethyl steht.

Insbesondere werden auch Verbindungen I bevorzugt, in denen ein Substituent R in 2-Position steht und n eine ganze Zahl von 1 bis 4, insbesondere 1 bis 3, bedeutet.

Außerdem werden Verbindungen I besonders bevorzugt, in denen n 2 oder 3 bedeutet und ein Substituent R in 2-Position steht.

Weiterhin werden Verbindungen I bevorzugt, in denen R für Fluor, Chlor, Brom, Cyano, C₁-C₆-Alkyl, C₁-C₆-Alkoxy, C₁-C₆-Alkoxycarbonyl, C₁-C₆-Alkylcarbonyl, C₁-C₆-Alkoximino-C₁-C₆-alkyl, C₁-C₆-Alkenyloximino-C₁-C₆-alkyl oder C₁-C₆-Alkinyloximino-C₁-C₆-alkyl steht.

Gleichermaßen besonders bevorzugt sind Verbindungen I, in denen R für Fluor, Chlor, Methyl, Trifluormethyl oder Methoxy steht.

Daneben werden Verbindungen I besonders bevorzugt, in denen Rₙ für 2-Chlor, 2-Fluor, 2,6-Difluor, 2-Methoxy, 2-Trifluormethyl, 2-Trifluormethyl,6-chlor, 2-Chlor,6-fluor, 2,4,6-Trifluor, 2,6-Difluor,4-methoxy oder Pentafluor steht.

Insbesondere werden Verbindungen I besonders bevorzugt, in denen Rₙ für 2-Chlor,6-fluor, 2,6-Difluor, 4-methoxy oder 2,4,6-Trifluor steht.

Außerdem werden Verbindungen IA besonders bevorzugt, in denen n, R und R¹ die Bedeutungen wie in Formel I haben:

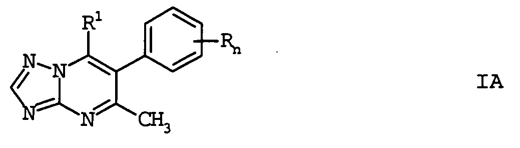

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceten, Phycomyceten* und *Basidiomyceten,* aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer; Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- *Alternaria*-Arten an Gemüse und Obst,
- *Botrytis cinerea* (Grauschimmel) an Erdbeeren, Gemüse, Zierpflanzen und Reben,
- *Cercospora arachidicola* an Erdnüssen,
- *Erysiphe cichoracearum* und *Sphaerotheca fuliginea* an Kürbisgewächsen,
- *Erysiphe graminis* (echter Mehltau) an Getreide,
- *Fusarium-* und *Verticillium*-Arten an verschiedenen Pflanzen,
- *Helminthosporium*-Arten an Getreide,
- *Mycosphaerella*-Arten an Bananen und Erdnüssen,
- *Phytophthora infestans* an Kartoffeln und Tomaten,
- *Plasmopara viticola* an Reben,
- *Podosphaera leucotricha* an Äpfeln,
- *Pseudocercosporella herpotrichoides* an Weizen und Gerste,
- *Pseudoperonospora*-Arten an Hopfen und Gurken,
- *Puccinia*-Arten an Getreide,
- *Pyricularia oryzae* an Reis,
- *Rhizoctonia*-Arten an Baumwolle, Reis und Rasen,
- *Septoria nodorum* an Weizen,
- *Uncinula necator* an Reben,
- *Ustilago*-Arten an Getreide und Zuckerrohr, sowie
- *Venturia*-Arten (Schorf) an Äpfeln und Birnen.

Die Verbindungen I eignen sich außerdem zur Bekämpfung von Schadpilzen wie *Paecilomyces variotii* im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2-Chlor-N-(4'-chlor-biphenyl-2-yl)-nicotinamid, 2,4-Dichlor-6-(o-chloranilino)-s-triazin, O,O-Diethyl-phthalimido-phosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsäure-diamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-for-mylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-ylethyl]-1H-1,2,4-triazol, N-(n-Propyl)-N-(2,4-,6-trichlorphen-oxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2-[6-(2-cyanophenoxy)-pyrimidin-4-yloxy]-phenyl}-3-methoxyacrylat, Methyl-E-methoxyimino-[α-(2-phenoxyphenyl)]-acetamid, Methyl-E-methoxyimino-[a-(2,5-dimethylphenoxy)-o-tolyl]-acetamid, Methyl-E-2-{2-[2-trifluorme-thylpyridyl-6-]oxymethyl]-phenyl}3-methoxyacrylat, (E,E)-Methoximino-{2-[1-(3-trifluormethylphenyl)-ethylidenaminooxymethyl]-phenyl}-essigsäuremethylester, Methyl-N-(2-{[1-(4-chlorphenyl)-1H-pyrazol-3-yl]oxymethyl}phenyl)N-methoxy-carbamat,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid, 3-(4-Fluorphenyl)-3-(3,4-dimethoxyphenyl)-acrylsäuremorpholid,
- sowie verschiedene Fungizide, wie Dodecylguanidinacetat, 1-(3-Brom-6-methoxy-2-methyl-phenyl)-1-(2,3,4-trimethoxy-6-methyl-phenyl)-methanon, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-(3,5-Dichlorphenyl)-5-methyl-5-methoxymethyl-1,3-oxazolidin- 2,4-dion, 3-(3,5-Dichlorphenyl)-1-isopropylcarbamoylhydantoin, N-(3,5-Dichlorphenyl)-1,2-dimethyl-cyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-a-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol, 5-Chlor-2-cyano-4-p-tolyl-imidazol-1-sulfonsäuredimethylamid, 3,5-Dichlor-N-(3-chlor-1-ethyl-1-methyl-2-oxo-propyl)-4-methyl-benzamid.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Angaben aufgeführt.

### Beispiel 1 Herstellung von 5,7-Dimethyl-6-phenyl-1,2,4-triazolo[1,5a]pyrimidin [I-1]

Eine Mischung von 0,84 g (10 mmol) 3-Aminotriazol und 1,8 g (28 mmol) 3-Phenyl-pentandion-(2,4) in 5 g Tributylamin wurde 8 Std. bei 140°C bis 180°C erhitzt. Nach Abkühlen auf 20 bis 25°C wurde der Niederschlag abfiltriert und mit Diisopropylether gewaschen. Man erhielt 0,3 g der Titelverbindung als farblose Kristalle. Das Filtrat wurde mit verd. Salzsäure extrahiert und die organische Phase wurde verworfen. Nach Neutralisation wurde die wässrige Phase mit Essigester extrahiert und eingeengt. Aus dem Rückstand wurde nach Chromatographie an Kieselgel (Cyclohexän/Essigester-Gemische) zusätzlich 0,5 g (insgesamt 36 %) der Titelverbindung als gelbliche Kristallmasse erhalten.
¹H-NMR (CDCl₃, δ in ppm): 8,5 (s, 1H); 7,5 (m, 3H); 7,2 (m, 2H); 2,6 (s, 3H); 2,45 (s, 3H).

### Beispiel 2 Herstellung von 7-Cyclohexyl-5-methyl-6-(2-Cl-,6-F-phenyl)-1,2,4-triazolo[1,5a]pyrimidin [I-4]

### a) 7-Cyclohexyl-5-(diethylmalon-2-yl)-6-(2-Cl-,6-F-phenyl)-1,2,4-triazolo[1,5a]pyrimidin

Eine Mischung von 30 g (0,18 mol) Malonsäurediethylester in 30 ml Acetonitril wurde mit 0,3 g (12 mmol) Natriumhydrid versetzt. Anschließend gab man 2,8 g (7,6 mmol) 5-Chlor-7-cyclohexyl-6-(2-Cl-,6-F-phenyl)-1,2,4-triazolo[1,5a]pyrimidin (WO-A 99/41255) hinzu und rührte die Reaktionsmischung ca. 5 Std. bei etwa 70°C. Dabei fielt das Natriumsalz des Produktes als gelber Festkörper aus, der abfiltriert und mit Acetonitril gewaschen wurde. Der Rückstand wurde mit etwas Kieselgur vermengt und mit einer Mischung aus verd. Salzsäure und Essigester gerührt. Die Acetonitril-Waschphase wurde ebenfalls mit verd. Salzsäure/Essigester gerührt. Die vereinigten Essigester-Phasen wurden getrocknet und eingeengt. Der Rückstand kristallisierte und wurde mit Diisopropylether digeriert. Man erhielt 1,4 g (38 %) der Titelverbindung als farblosen Festkörper.
¹H-NMR (CDCl₃, δ in ppm): 8,55 (s, 1H); 7,5 (m, 2H); 7,2 (t, 1H); 4,6 (s, 1H); 4,0 - 4,4 (m, 4H); 2,3 -2,9 (m, 3H); 1,6 - 1,9 ( m, 5H); 1,05 - 1,4 (m, 9H).

### b) 7-Cyclohexyl-5-methyl-6-(2-Chlor-,6-fluor-phenyl)-1,2,4-triazolo[1,5a]pyrimidin

Eine Mischung von 1,1 g (2,2 mmol) 7-Cyclohexyl-5-(diethylmalon-2-yl)-6-(2-Cl-,6-F-phenyl)-1,2,4-triazolo[1,5a]pyrimidin (Beispiel 2a) in 10 ml konz. Salzsäure wurde 2 Stunden bei 80 bis 90°C gerührt. Nach Abkühlen auf 20 bis 25°C wurde mit Wasser verdünnt und die wässrige Phase mit CH₂Cl₂ extrahiert. Die vereinigten organischen Phasen wurden mit Natriumcarbonat-Lsg. gewaschen, getrocknet und eingeengt. Der Rückstand kristallisierte und wurde mit Diisopropylether digeriert. Man erhielt 0,5 g (66 %) der Titelverbindung als farblosen Festkörper vom Fp. 182 bis 184°C.
¹H-NMR (CDCl₃, δ in ppm): 8,5 (s, 1H); 7,5 (m, 2H); 7,2 (t, 1H); 2,2 - 2,9 (m, 3H); 2,6 (s, 3H); 1,6 - 1,9 (m, 5H); 1,15 - 1,4 (m, 3H).

### Beispiel 3 Herstellung von 7-Isobutyl-5-ethyl-6-(2-Cl-,6-F-phenyl)-1,2,4-triazolo[1,5a]pyrimidin [I-14]

Durch eine Mischung von 1,7 g (5 mmol) 5-Cl-7-isobutyl-6-(2-Cl-,6-F-phenyl)-1,2,4-triazolo[1,5a]pyrimidin (WO-A 99/41255) in 40 ml Tetrahydrofuran wurde ca. 15 min ein Argonstrom geleitet. Anschließend gab man 0,15 g (0,25 mmol) (1,3-Bis-(diphenylphosphino)-propan)-nickel-II-chlorid und 0,75 g (6 mmol) Diethylzink hinzu und rührte ca. 3 Std. bei 20 bis 25°C. Die Reaktionsmischung wurde mit Wasser verdünnt und mit CH₂Cl₂ extrahiert. Die vereinigten organischen Phasen wurden getrocknet und eingeengt.

Aus dem Rückstand erhielt man nach Chromatographie an Kieselgel (RP 18) mit Cyclohexan/Essigester-Gemischen 0,2 g (12 %) der Titelverbindung als farblosen Festkörper vom Fp. 106 - 108°C.
¹H-NMR (CDCl₃, δ in ppm): 8,5 (s, 1H); 7,5 (m, 1H); 7,45 (d, 1H); 7,2 (t, 1H); 3,05 (dd, 1H); 2,7 (m, 3H); 2,3 (m, 1H); 1,25 (t, 3H); 0,9 (d, 3H); 0,8 (d, 3H).

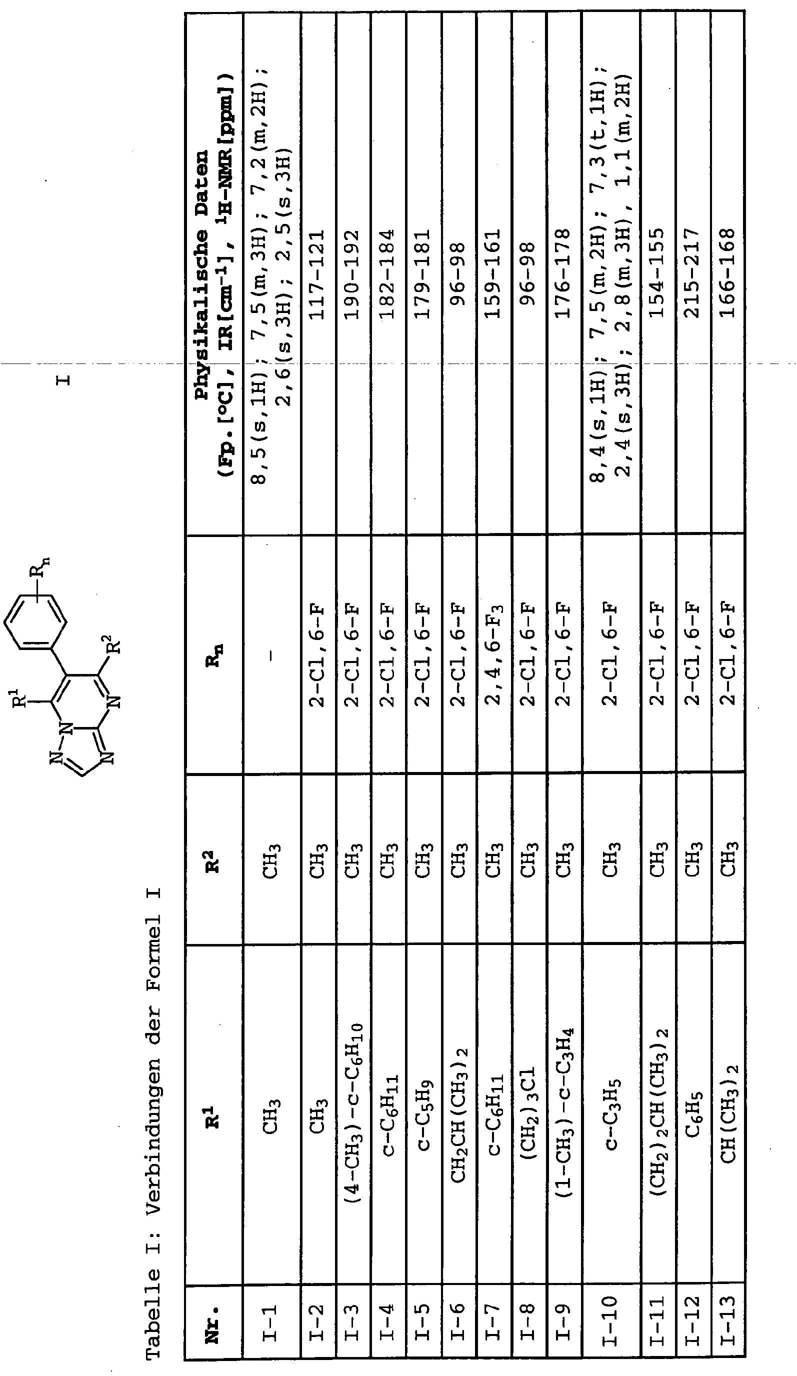

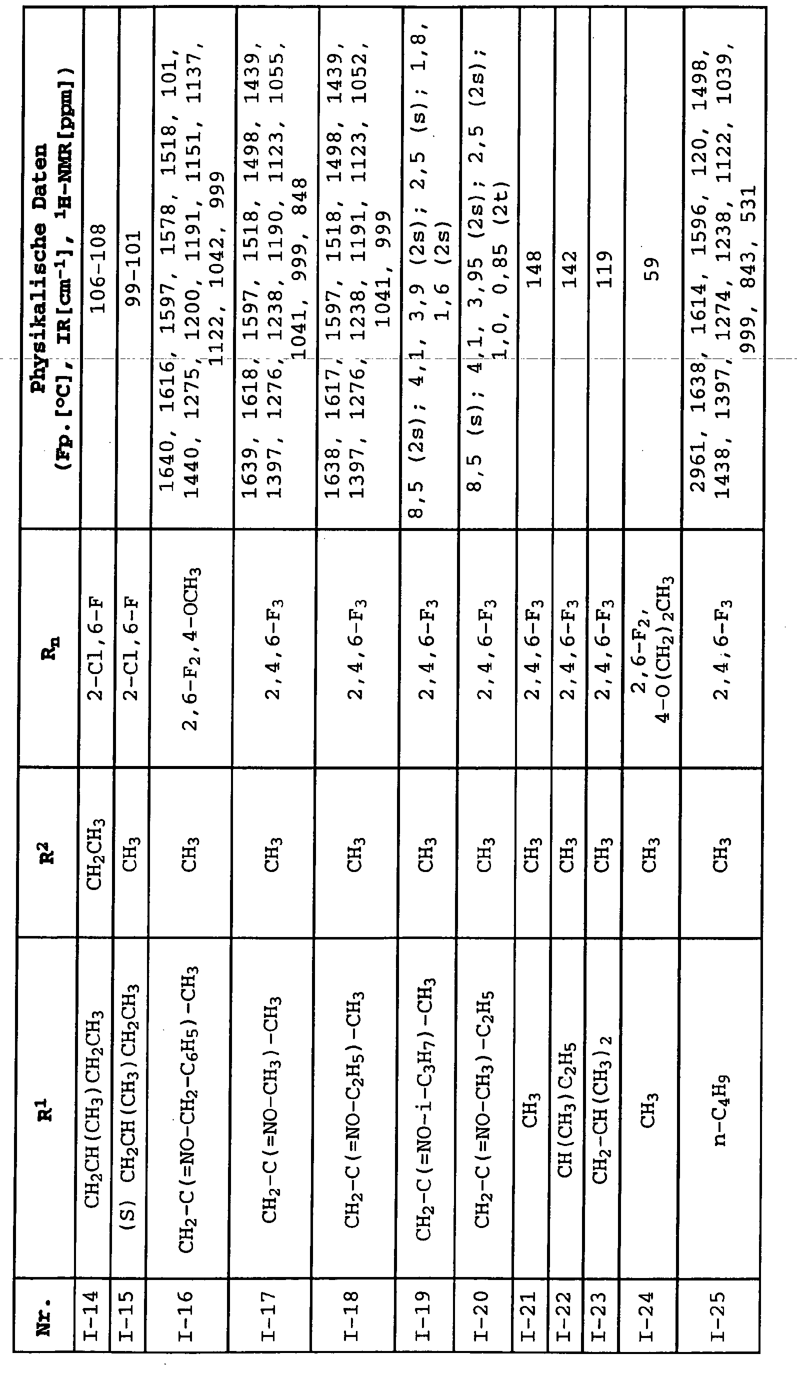

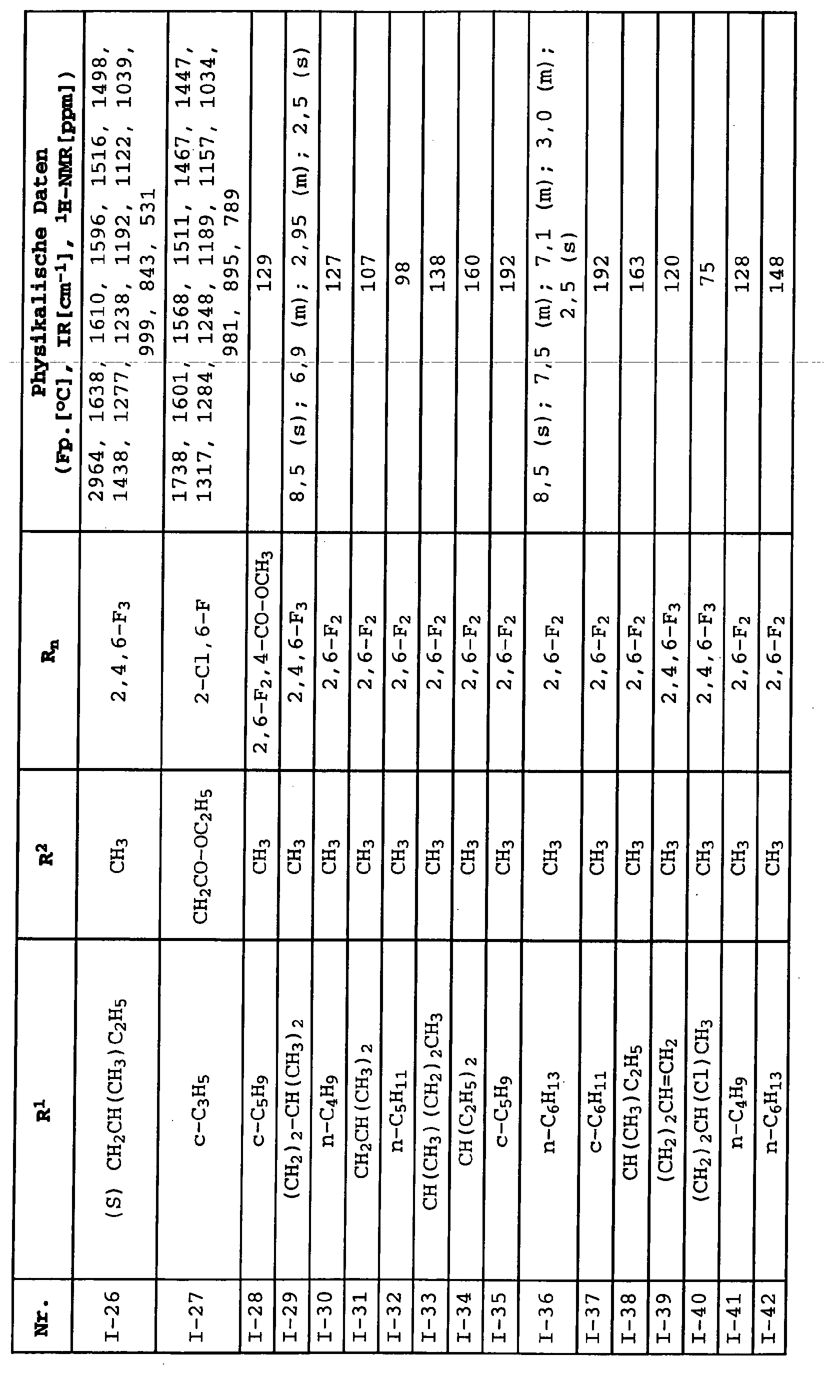

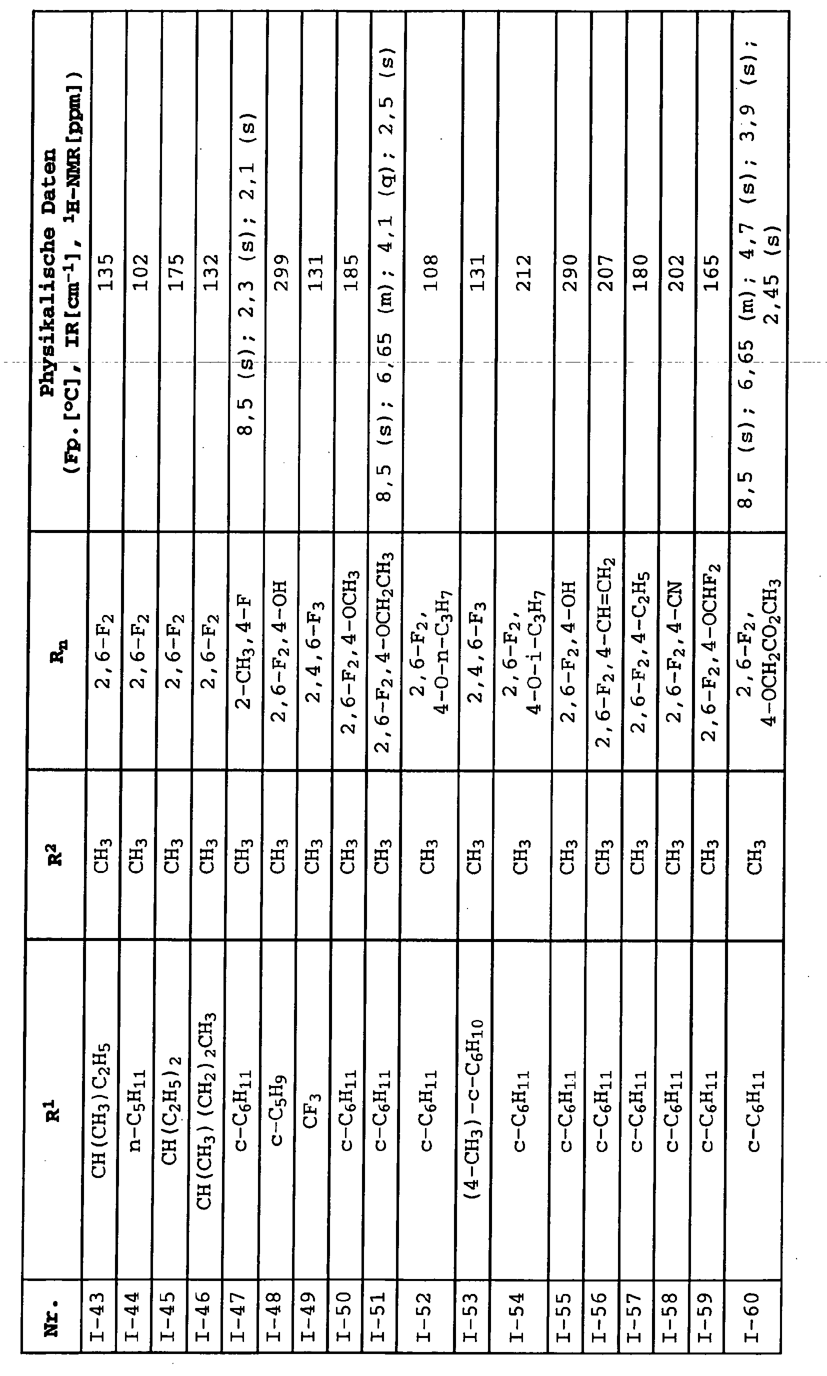

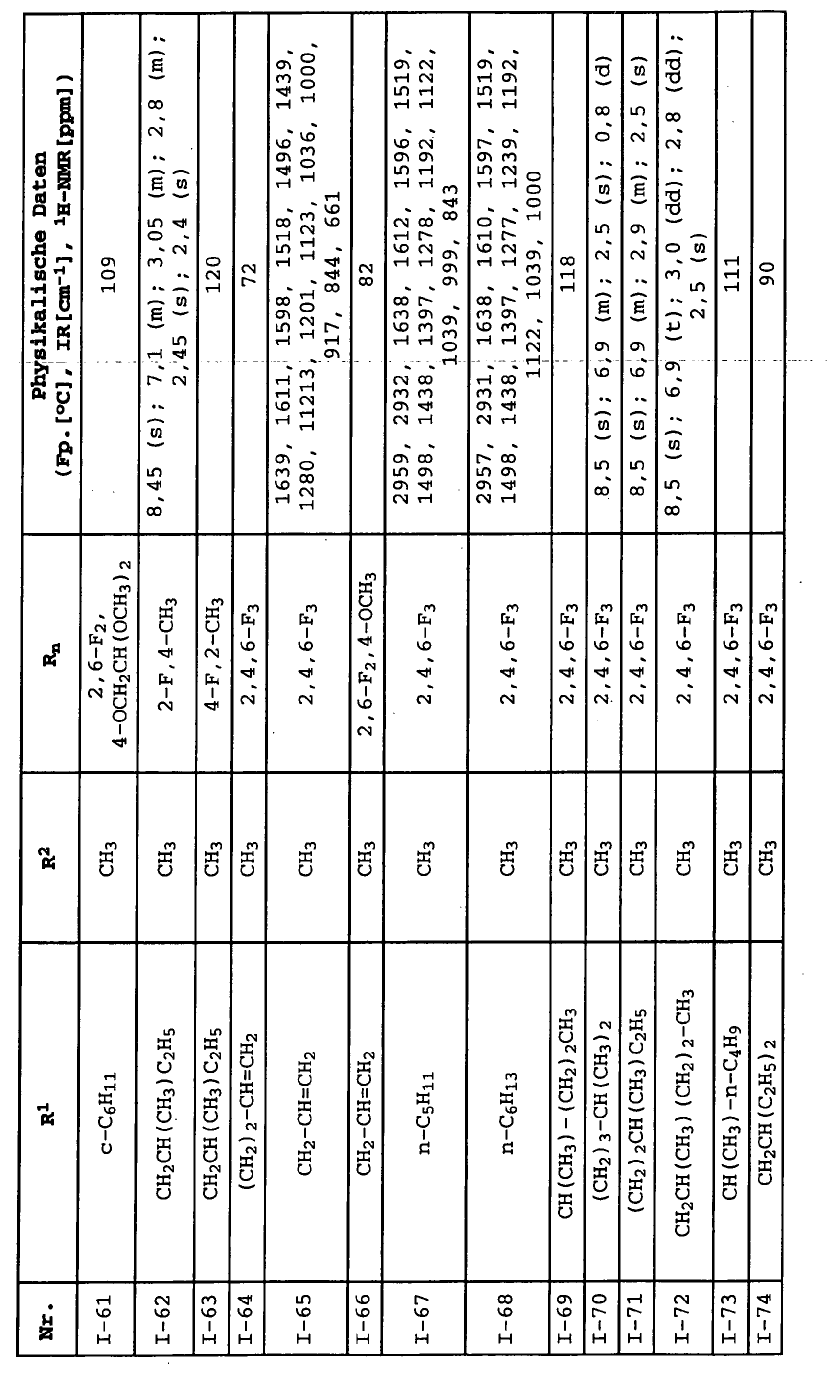

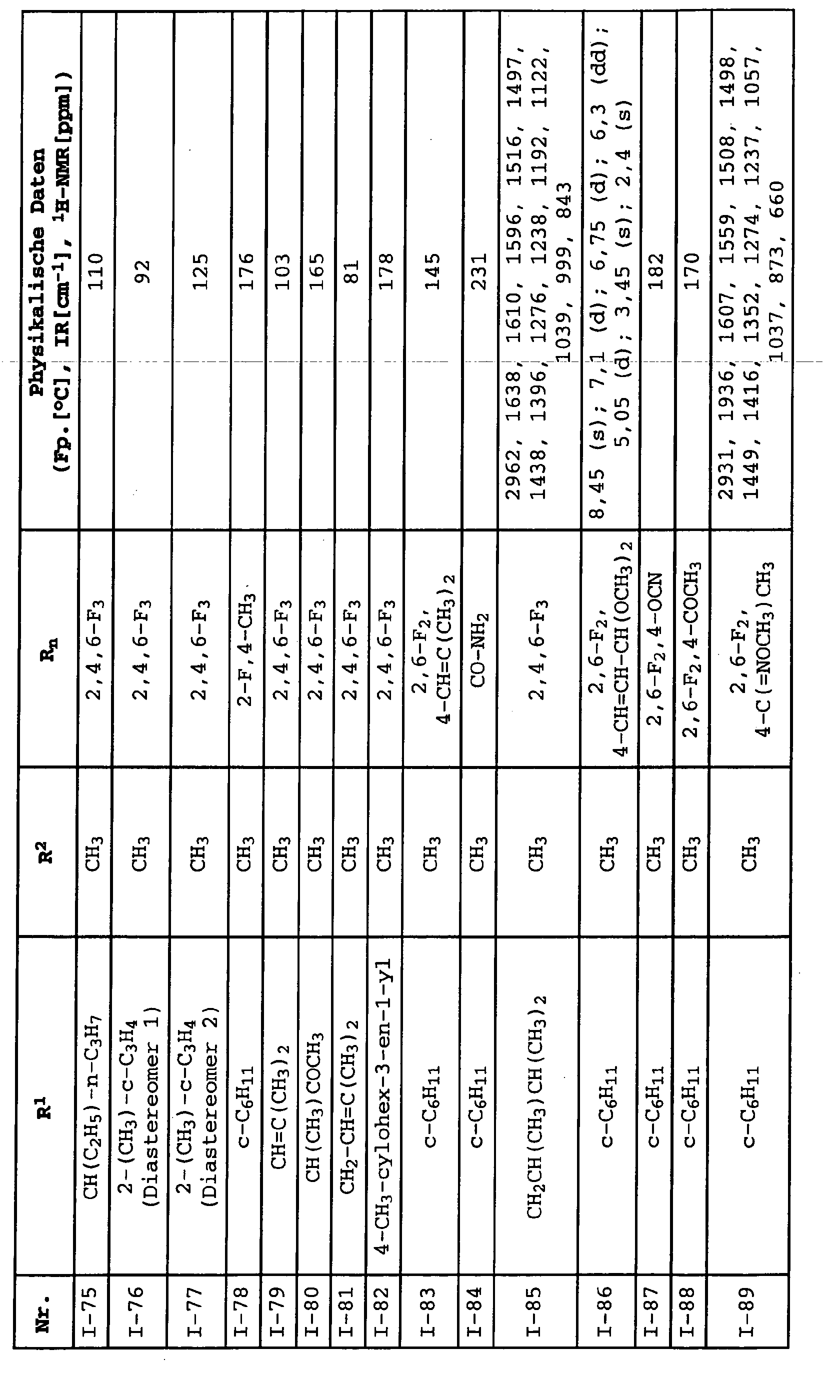

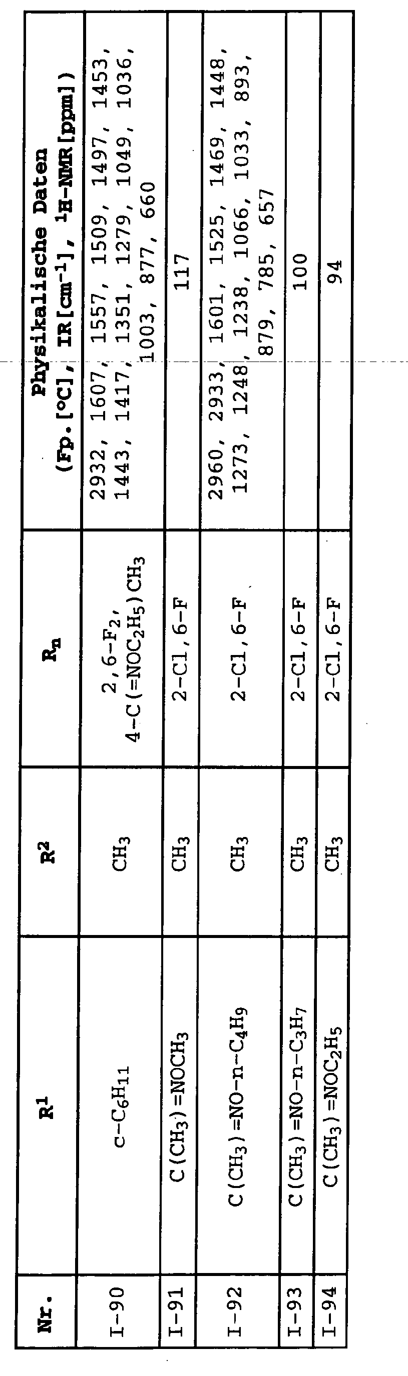

### Beispiele für die Wirkung gegen Schadpilze

Die fungizide Wirkung der Verbindungen der allgemeinen Formel I ließ sich durch die folgenden Versuche zeigen:

Die Wirkstoffe wurden getrennt oder gemeinsam als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

Als Vergleichswirkstoffe dienten die aus WO-A 99/41255 bekannten Verbindungen A bis F:

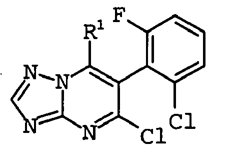

| **Nr.** | **bekannt aus** | **R**^{**1**} |
|---|---|---|
| A | WO-A 99/41255, Nr.2c | 4-CH₃-C-C₆H₁₀ |
| B | WO-A 99/41255, Nr.26 | CH₂CH(CH₃)₂ |
| C | WO-A 99/41255, Nr.27 | CH(CH₃)₂ |
| D | WO-A 99/41255, Nr.29 | c-C₅H₉ |
| E | WO-A 99/41255, Nr.30 | c-C₇H₁₃ |
| F | WO-A 99/41255, Nr.31 | C₆H₅ |

### Anwendungsbeispiel 1 - Wirksamkeit gegen Alternaria solani an Tomaten

Blätter von Topfpflanzen der Sorte "Große Fleischtomate St. Pierre" wurden mit einer wäßrigen Suspension, die aus einer Stammlösung aus 10 % Wirkstoff, 63 % Cyclohexanon und 27 % Emulgiermittel angesetzt wurde, bis zur Tropfnässe besprüht. Am folgenden Tag wurden die Blätter mit einer wäßrigen Zoosporenaufschwemmung von *Alternaria solani* in 2 % Biomalzlösung mit einer Dichte von 0,17 x 10⁶ Sporen/ml infiziert. Anschließend wurden die Pflanzen in einer wasserdampfgesättigten Kammer bei Temperaturen zwischen 20 und 22°C aufgestellt. Nach 5 Tagen hatte sich die Krautfäule auf den unbehandelten, jedoch infizierten Kontrollpflanzen so stark entwickelt, daß der Befall visuell in % ermittelt werden konnte.

In diesem Test zeigten die mit 63 ppm der Wirkstoffe I-3 bis I-8, I-11 bis I-15, I-18, I-20, I-22, I-23, I-25, I-26, I-28 bis I-32, I-35 bis I-37, I-40, I-41, I-42, I-44, I-47, I-48, I-50 bis I-I-54, I-56, I-58, I-59, I-61, I-62, I-64 und I-67 bis I-74 der Tabelle I behandelten Pflanzen maximal 10 % Befall, während die mit mit 63 ppm der Vergleichswirkstoffe C und D behandelten zu mindestens 80 % und die unbehandelten Pflanzen zu 100 % befallen waren.

### Anwendungsbeispiel 2 - Protektive Wirksamkeit gegen Gurkenmehltau

Blätter von in Töpfen gewachsenen Gurkenkeimlingen der Sorte "Chinesische Schlange" wurden im Keimblattstadium mit wäßriger Wirkstoffaufbereitung, die mit einer Stammlösung aus 10 % Wirkstoff, 63 % Cyclohexanon und 27 % Emulgiermittel angesetzt wurde, bis zur Tropfnässe besprüht. 20 Stunden nach dem Antrocknen des Spritzbelages wurden die Pflanzen mit einer wäßrigen Sporensuspension des Gurkenmehltaus (*Sphaerotheca fuliginea)* inokuliert. Anschließend wurden die Pflanzen im Gewächshaus bei Temperaturen zwischen 20 und 24°C und 60 bis 80 % relativer Luftfeuchtigkeit für 7 Tage kultiviert. Dann wurde das Ausmaß der Mehltauentwicklung visuell in %-Befall der Keimblattfläche ermittelt.

In diesem Test zeigten die mit 63 ppm der Wirkstoffe I-3 bis I-9, I-11 bis I-15, I-17, I-18, I-20, I-22, I-23, I-25, I-26, I-28 bis I-32, I-34, I-35, I-37, I-38, I-40, I-41, I-43, I-46, I-47, I-52, I-53, I-58, I-63, I-64, I-66 bis I-75, I-93 und I-94 der Tabelle I behandelten Pflanzen keinen oder bis maximal 10 % Befall, während die mit mit 63 ppm der Vergleichswirkstoffe A bis F behandelten zu mindestens 60 % und die unbehandelten Pflanzen zu 100 % befallen waren.

### Anwendungsbeispiel 3 - Protektive Wirksamkeit gegen die Netzflekkenkrankheit der Gerste (Pyrenophora teres)

Blätter von in Töpfen gewachsenen Gerstenkeimlingen der Sorte "Igri" wurden mit wäßriger Wirkstoffaufbereitung, die aus einer Stammlösung bestehend aus 10 % Wirkstoff, 63 % Cyclohexanon und 27 % Emulgiermittel angesetzt wurde, bis zur Tropfnässe besprüht und 24 Stunden nach dem Antrocknen des Spritzbelages mit einer wäßrigen Sporensuspension von *Pyrenophora teres,* dem Erreger der Netzfleckenkrankheit inokuliert. Anschließend wurden die Versuchspflanzen im Gewächshaus bei Temperaturen zwischen 20 und 24°C und 95 bis 100 % relativer Luftfeuchtigkeit aufgestellt. Nach 6 Tagen wurde das Ausmaß der Krankheitsentwicklung visuell in % Befall der gesamten Blattfläche ermittelt.

In diesem Test zeigten die mit 63 ppm der Wirkstoffe I-3 bis I-8, I-11, I-12, I-14, I-15, I-18, I-22, I-23, I-25, I-26, I-28 bis I-32, I-35, I-36, I-37, I-40 bis I-44, I-47, I-50 bis I-53, I-58, I-61 bis I-64, I-66 bis I-74 und I-77 der Tabelle I behandelten Pflanzen maximal 20 % Befall, während die mit mit 63 ppm der Vergleichswirkstoffe B, D und F behandelten zu mindestens 60 % und die unbehandelten Pflanzen zu 100 % befallen waren.

## Patentansprüche

1. Triazolopyrimidine der Formel I 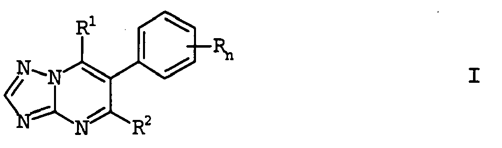 in der der Index und die Substituenten folgende Bedeutung haben:
n 0 oder eine ganze Zahl von 1 bis 5;
R Halogen, Cyano, Hydroxy, Cyanato (OCN), C₁-C₈-Alkyl, C₂-C₁₀-Alkenyl, C₂-C₁₀-Alkinyl, C₁-C₆-Halogenalkyl, C₂-C₁₀-Halogenalkenyl, C₁-C₆-Alkoxy, C₂-C₁₀-Alkenyloxy, C₂-C₁₀-Alkinyloxy, C₁-C₆-Halogenalkoxy, C₃-C₆-Cycloalkyl, C₃-C₆-Cycloalkenyl, C₃-C₆-Cycloalkoxy, C₁-C₈-Alkoxycarbonyl, C₂-C₁₀-Alkenyloxycarbonyl, C₂-C₁₀-Alkinyloxycarbonyl, Aminocarbonyl, C₁-C₈-Alkylaminocarbonyl, Di-(C₁-C₈-)alkylaminocarbonyl, C₁-C₈-Alkoximinoalkyl, C₂-C₁₀-Alkenyloximinocarbonyl, C₂-C₁₀-Alkinyloximinoalkyl, C₁-C₈-Alkylcarbonyl, C₂-C₁₀-Alkenylcarbonyl, C₂-C₁₀-Alkinylcarbonyl, C₃-C₆-Cycloalkylcarbonyl, oder ein fünf- bis zehngliedriger gesättigter, partiell ungesättigter oder aromatischer Heterocyclus, enthaltend ein bis vier Heteroatome aus der Gruppe O, N oder S;
R¹ ein fünf- bis zehngliedriger gesättigter, partiell ungesättigter oder aromatischer Heterocyclus, ausgewählt aus 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-3-yl, 3,4-Dihydropyrazol-4-yl, 3,4-Dihydropyrazol-5-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl; 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl; wobei in den 5-Ring Heteroarylgruppen zwei benachbarte Kohlenstoffringglieder oder ein Stickstoff- und ein benachbartes Kohlenstoffringglied durch eine Buta-1,3-dien-1,4-diylgruppe verbrückt sein können;
wobei R und/oder R¹ partiell oder vollständig halogeniert oder durch eine bis vier gleiche oder verschiedene Gruppen R^{a} substituiert sein können:
R^{a} Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy, C₃-C₆-Alkinyloxy, C₃-C₆-Cycloalkyl, C₁-C₈-Alkoximino, C₂-C₁₀-Alkenyloximino, C₂-C₁₀-Alkinyloximino, Aryl-C₁-C₈-alkyloximino, C₂-C₁₀-Alkinyl, C₂-C₁₀-Alkenyloxycarbonyl, C₂-C₁₀-Alkinyloxycarbonyl, Phenyl, Naphthyl, fünf- bis zehngliedriger gesättigter, partiell ungesättigter oder aromatischer Heterocyclus, enthaltend ein bis vier Heteroatome aus der Gruppe O, N oder S,
wobei diese aliphatischen, alicyclischen oder aromatischen Gruppen ihrerseits partiell oder vollständig halogeniert sein oder eine bis drei Gruppen R^{b} tragen können:
R^{b} Halogen, Cyano, Nitro, Hydroxy, Mercapto, Amino, Carboxyl, Aminocarbonyl, Aminothiocarbonyl, Alkyl, Haloalkyl, Alkenyl, Alkenyloxy, Alkinyloxy, Alkoxy, Halogenalkoxy, Alkylthio, Alkylamino, Dialkylamino, Formyl, Alkylcarbonyl, Alkylsulfonyl, Alkylsulfoxyl, Alkoxycarbonyl, Alkylcarbonyloxy, Alkylaminocarbonyl, Dialkylaminocarbonyl, Alkylaminothiocarbonyl, Dialkylaminothiocarbonyl, wobei die Alkylgruppen in diesen Resten 1 bis 6 Kohlenstoffatome enthalten und die genannten Alkenyl- oder Alkinylgruppen in diesen Resten 2 bis 8 Kohlenstoffatome enthalten;
und/oder einen bis drei der folgenden Reste:
Cycloalkyl, Cycloalkoxy, Heterocyclyl, Heterocyclyloxy, wobei die cyclischen Systeme 3 bis 10 Ringglieder enthalten; Aryl, Aryloxy, Arylthio, Aryl-C₁-C₆-alkoxy, Aryl-C₁-C₆-alkyl, Hetaryl, Hetaryloxy, Hetarylthio, wobei die Arylreste vorzugsweise 6 bis 10 Ringglieder, die Hetarylreste 5 oder 6 Ringglieder enthalten, wobei die cyclischen Systeme partiell oder vollständig halogeniert oder durch Alkyl- oder Haloalkylgruppen substituiert sein können; und
R² C₁-C₄-Alkyl, C₂-C₄-Alkenyl oder C₂-C₄-Alkinyl, die durch Halogen, Cyano, Nitro, C₁-C₂-Alkoxy oder C₁-C₄-Alkoxycarbonyl substituiert sein können.

2. Triazolopyrimidine der Formel I gemäß Anspruch 1, in der der Index und die Substituenten folgende Bedeutung haben:
n 0 oder eine ganze Zahl von 1 bis 5;
R Halogen, Cyano, C₁-C₆-Alkyl, C₂-C₁₀-Alkenyl, C₂-C₁₀-Alkinyl, C₁-C₆-Halogenalkyl, C₂-C₁₀-Halogenalkenyl, C₁-C₆-Alkoxy, C₂-C₁₀-Alkenyloxy, C₂-C₁₀-Alkinyloxy, C₁-C₆-Halogenalkoxy, C₃-C₆-Cycloalkyl, C₃-C₆-Cycloalkenyl, C₃-C₆-Cycloalkoxy oder ein fünf- bis zehngliedriger gesättigter, partiell ungesättigter oder aromatischer Heterocyclus, enthaltend ein bis vier Heteroatome aus der Gruppe O, N oder S;
R¹ ein fünf- bis zehngliedriger gesättigter, partiell ungesättigter oder aromatischer Heterocyclus, enthaltend ein bis vier Heteroatome aus der Gruppe O, N oder S,
wobei R¹ partiell oder vollständig halogeniert oder durch eine bis vier gleiche oder verschiedene Gruppen R^{a} substituiert sein kann:
R^{a} Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy, C₃-c₆-Alkinyloxy, C₃-C₆-Cycloalkyl, Phenyl, Naphthyl, fünf- bis zehngliedriger gesättigter, partiell ungesättigter oder aromatischer Heterocyclus, enthaltend ein bis vier Heteroatome aus der Gruppe O, N oder S,
wobei diese aliphatischen, alicyclischen oder aromatischen Gruppen ihrerseits partiell oder vollständig halogeniert sein oder eine bis drei Gruppen R^{b} tragen können, und
R² C₁-C₄-Alkyl, das durch Halogen, Cyano, Nitro oder C₁-C₂-Alkoxy substituiert sein kann.

3. Verfahren zur Herstellung der Verbindungen der Formel I gemäß Ansprüchen 1 bis 3 durch Umsetzung von 5-Aminotriazol der Formel II 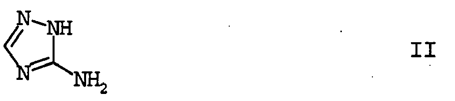 mit Dicarbonylverbindungen der Formel III.

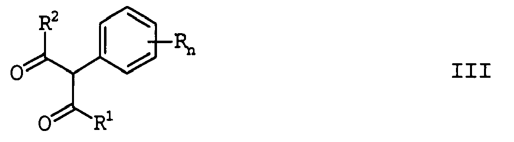

4. Verfahren zur Herstellung der Verbindungen der Formel I' 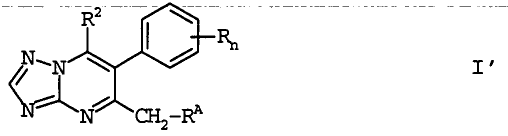
wobei n, R und R¹ die in Ansprüchen 1 bis 3 gegebene Bedeutung haben und R^{A} Wasserstoff oder C₁-C₃-Alkyl, das gemäß Ansprüchen 1 bis 3 substituiert sein kann, bedeutet, durch Umsetzung von Halogenverbindungen der Formel IV 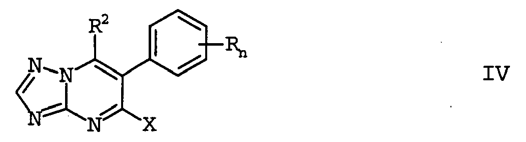 in der X für Halogen steht, mit substituierten Malonsäureestern der Formel V, 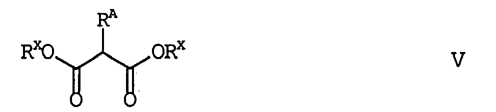 in der R^{X} für C₁-C₄-Alkyl, Allyl, Phenyl oder Benzyl steht, zu Verbindungen der Formel VI 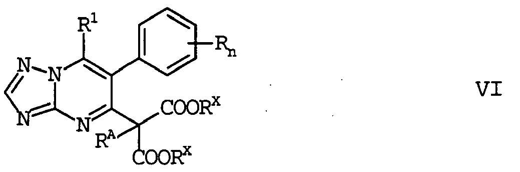 anschließender Verseifung von VI zu der Säure VIa und Decarboxilierung von VIa

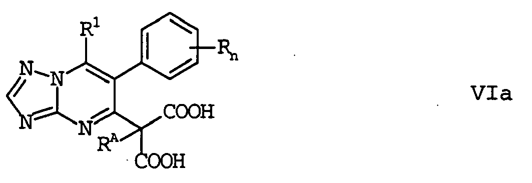

5. Zur Bekämpfung von Schadpilzen geeignetes Mittel, enthaltend einen festen oder flüssigen Trägerstoff und eine Verbindung der Formel I gemäß Anspruch 1.

6. Verwendung der Verbindungen I gemäß Anspruch 1 zur Herstellung eines zur Bekämpfung von Schadpilzen geeigneten Mittels.

7. Verfahren zur Bekämpfung von Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der Formel I gemäß Anspruch 1 behandelt.
